# EUROPEAN PATENT APPLICATION

(11) **EP 2 835 145 A1**
(43) Date of publication of application: **11.02.2015**
(21) Application number: 14179614.4
(22) Date of filing: 03.08.2014
(51) Int. Cl.: A61M 5/32

(54) **Safety hypodermic syringe**

(30) Priority: 05.08.2013 GB 201314004
(71) Applicant: Dean, Mohamed Salim, Mitcham, Surrey CR4 2PE (GB)
(72) Inventor: Dean, Mohamed Salim, Mitcham, Surrey CR4 2PE (GB)
(74) Representative: Pedder, James Cuthbert

(57) **Abstract**

1. A safety hypodermic syringe (1) comprises a barrel (3) having a plunger operated piston therein and a hollow spigot (11) extending from one end (7) of the barrel (3) and connected to the interior of the barrel (3) An hollow needle (13) is detachably attached to the spigot (11) by means of a cup portion (15) at the end of the needle (13) away from the point (18), the spigot (11) having a reduced diameter portion at its distal end for receipt of the needle cup portion (15) so that the cup portion (15) lies substantially flush with the remainder of the spigot (11). A cap (31) is detachably fitted to the said one end (7) of the barrel (3) so as to cover the needle (13). The inner diameter (D) of the cap (31) is at least one sixth larger than the outer diameter (d) of the needle cup portion (15) and/or the cap (31) has a length (L) measured from the inner end (16) of the needle (13) adjacent to the needle cup portion (15) to the opposite end of the cap (31), which is at least twice the length (I) of the needle (13) being the length of the needle (13) beyond the cup portion (15).

## Description

This invention relates to a safety hypodermic syringe.

In use of hypodermic syringes a sterile needle is attached to the injection end of the syringe and this is then used to load the syringe. Once loaded, the needle on the syringe is covered by a relatively small cap to prevent contact with the needle.

However, problems have arisen in the use of such caps in that. when manipulating the cap. the users fingers must, of necessity, be in close proximity to the needle and accidents resulting from this are endemic.

The invention seeks to reduce or obviate the above problems and render it considerably more difficult for accidents to happen.

According to a first aspect of the invention, there is provided a safety hypodermic syringe comprising a barrel having a plunger operated piston therein, a hollow spigot extending from one end of the barrel and connected to the interior of the barrel, an hollow needle detachably attached to the spigot by means of a cup portion at the end of the needle away from the point, the spigot having a reduced diameter portion at its distal end for receipt of the needle cup so that the cup portion lies substantially flush with the remainder of the spigot and a cap detachably fitted to the said one end of the barrel so as to cover the needle, wherein the cap has a length measured from the inner end of the needle adjacent to the needle cup portion to the opposite end of the cap, which is at least twice the length of the needle being the length of the needle beyond the cup portion.

According to a second aspect of the invention, there is provided a safety hypodermic syringe comprising a barrel having a plunger operated piston therein, a hollow spigot extending from one end of the barrel and connected to the interior of the barrel, an hollow needle detachably attached to the spigot by means of a cup portion at the end of the needle away from the point, the spigot having a reduced diameter portion at its distal end for receipt of the needle cup portion so that the cup portion lies substantially flush with the remainder of the spigot and a cap detachably fitted to the said one end of the barrel so as to cover the needle, wherein the inner diameter of the cap is at least one sixth larger than the outer diameter of the needle cup portion.

Preferably the two aspects of the invention are combined.

The inner diameter of the cap may be at least 7 mm, preferably at least 10 mm and most preferably at least 12 mm.

The outer diameter of the spigot may be substantially the same as the inner diameter of the cap.

The cap may have an increased diameter at its open end to a diameter substantially the same as the outer diameter of the barrel whereby the open end of the cap can be seated on the end of the barrel.

The length of the cap may be at least twice the length of the longest needle usable in the syringe. The length of the cap may be at least 30 mm..

The length of the cap may be at least 44 mm or preferably at least 70 mm.

The barrel and cap may have cooperating locking means whereby the cap can be locked in position relative to the barrel.

The spigot or the barrel may have a radially outwardly extending projection which cooperates with an axial slot in the cap extending in the axial direction of the cap from the open end thereof. A circumferential slot may be provided extending from the axial slot and into which the projection can be seated to stop removal of the cap. A second circumferential slot may be provided parallel to the first circumferential slot so that the cap is lockable in two positions.

The invention will now be described in greater detail, way of example with reference to the drawings, in which:-
Figure 1 is a schematic overall side section of a known syringe to which the invention is applied.
Figure 2 is a schematic sectional view of one end of the syringe of figure 1 carrying a cap over the needle in known form.
Figure 3 is a schematic view similar to figure 2 but showing a cap arrangement in accordance with a first embodiment of the invention,
Figure 4 is a schematic view similar to figure 2 but showing a cap arrangement in accordance with a second embodiment of the invention, and
Figure 5 is a schematic external view of the end of the syringe having a two position locking mechanism for the cap.

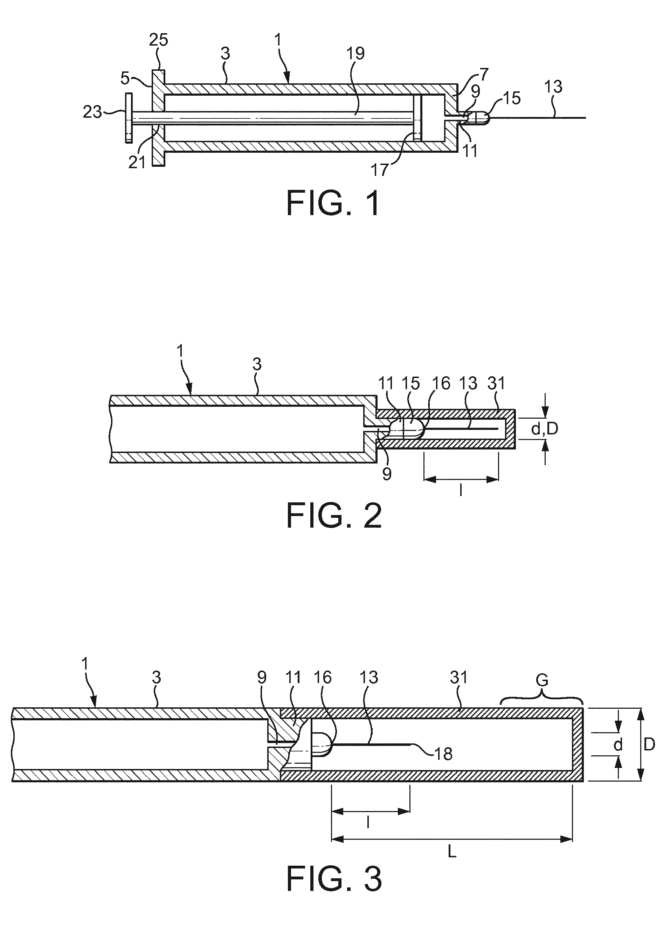

Referring firstly to figure 1, there is shown schematically the construction of a standard syringe of generally plastics construction. The syringe 1 comprises a hollow barrel 3 for receiving fluid to be injected. The barrel 3 is closed at one end by an end cap 5 which is attached to the barrel 3 such as by a screw arrangement. Where the syringe 1 is to be disposable, the cap 5 may be attached using a suitable adhesive. The other end 7 of the barrel is substantially closed but for a central channel 9 which permits egress of fluid from the barrel 3 or vice versa. The end 7 is provided with an hollow central cylindrical spigot 11 extending from the end 7 and carrying the channel 9. The distal end of the spigot 11 has a reduced diameter portion (not shown) to receive a detachable hollow metal needle 13 by means of a cup portion 15 on the end thereof which is pushed or screwed thereonto to secure the needle 13 in position. The cup portion 15 will have an outer diameter substantially the same as the outer diameter of the rest of the spigot 11 so as to be substantially flush therewith. Within the barrel 3 is located a piston 17 which is movable up and down the barrel 3 by means of a piston rod 19 which passes through an aperture 21 in the cap 5. The rod 19 has a head 23 on its end to facilitate its operation and the cap 5 extends outwards of the barrel 3 as shown at 25 for a similar reason.

In use , the syringe 1 can be used to obtain liquid from an ampoule or other liquid holding device so that it can be filled with fluid and thereafter the end of the needle 13 can be inserted into a patient to inject the liquid into them.

It will be appreciated that not only is the needle very sharp but it also needs to be kept in a sterile condition. For this reason, it is usually provided with a cap which covers the needle and is detachably secured to the spigot 11. Such a known arrangement is shown in figure 2 which shows one end of the known syringe of figure 1 fitted with a cap 31. The cap 31 has an inner diameter D which is substantially the same as the outer diameter d of the spigot 11, enabling the cap 31 to be a push fit thereon, and has a length which is just slightly longer than the length I of the needle 13.

However, removal of the cap 31 from the syringe 1 can be a risky process. The fingers of the hand removing the cap 31 will at all times be in close proximity to the needle 13 and this will mean that there is a danger of the fingers touching the needle 13 and in the worst scenario, being pricked by the needle 13. Thus, in accordance with one embodiment of the invention as seen in figure 3, the cap 31 has a length L which is at least twice the length I of the needle 13 and has an inner diameter D which is at least one sixth larger than the diameter d of the needle cup 15 which is the same diameter as the original the spigot 11 in figure 2. However, it is preferred that the diameter D is twice the diameter of the original spigot 11.

It will be understood that the length L of the cap 31 is measured from the end 16 of the needle 13 adjacent the needle cup 15 to the opposite inner end of the cap 31. The length I of the needle is defined as the length of the needle 13 from its end 16 adjacent to the needle cup 15 to the point 18 of the needle 13.

The arrangement shown in figure 3 will mean providing a new barrel 3 with the spigot 11 having an outer diameter d the same as the inner diameter D of the cap 31 Thus, if the cap 31 is gripped at its outer end in the region indicated as G, the fingers of the hand gripping the cap 31 will at no time approach the needle during the removal of the cap 31.

While it has been stated that the length L of the cap 31 is at least double the length I of the needle, it will be appreciated that various lengths of needles 13 can be used with the same barrel 3, and therefore it is preferred that the cap should have a length L which is double the length I of the longest needle usable with the syringe 1. Thus with the usual dimensions of the needle lengths as 15 mm, 22 mm and 35 mm , the minimum length L of the cap 31 will be 30 mm or 44 mm and the preferred length L of the cap 31 will be 70 mm .Lengths L intermediate the minimum and preferred lengths can be used depending on the needle size.

While there are a number of different sizes of syringe, these are usually usable with the same needles. The usual syringe sizes are 5 ml and 10 mls for the general plasti8cs syringes and 1.8 mls and 2.2 mls. for dental syringes. Thus the normal diameter d of the spigot 11 to allow attachment of the needle is 6 mm, the minimum diameter D of the inside of the cap 31 will be 7 mm but the preferred diameter D is 10 mm and may be even larger such as 12 mm, i.e. twice the diameter d.

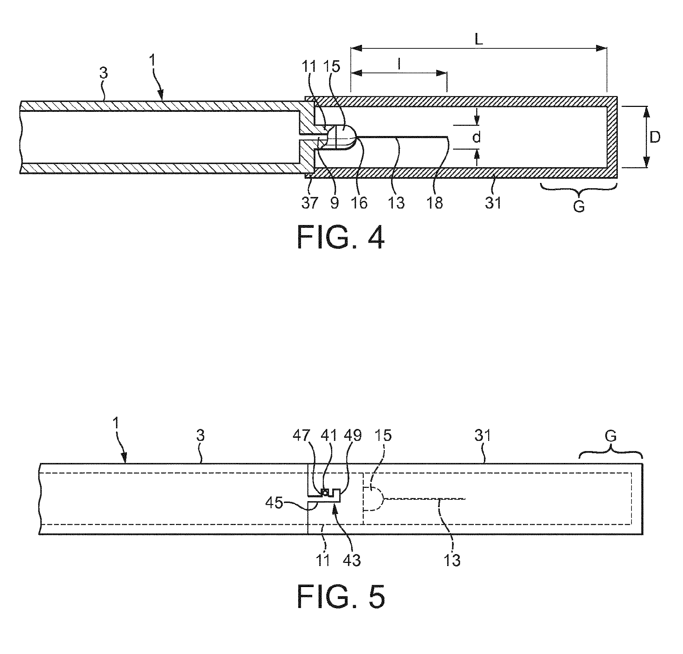

A second embodiment of the invention is shown in figure 4. This embodiment provides for a situation where only a new cap 31 is needed while retaining the original barrel 3 and spigot 11. Thus, in this case, the cap 31 is provided with an increased inner diameter portion 37 which is substantially the same as the outer diameter of the barrel 3 so that the cap 31 can be a push fit on the end of the barrel 3. The construction of the barrel 3 and spigot 11 remain the same as in figure 2.

Figure 5 shows a further safety feature in which the cap 31 can be locked in position relatively to the barrel. The locking arrangement shown here is particularly designed for the embodiment of figure 3. A radially outwardly extending projection 41 is provided on the exterior of the enlarged spigot 11 and this projection cooperates with a slot arrangement 43 cut into the wall of the cap 31. This slot arrangement comprises a first axial slot 45 extending in the axial or longitudinal direction of the cap 31 and two circumferential slots 47 and 49 extending circumferentially from the axial slot 45.

In this figure, the projection 41 is located in the first circumferential slot 47 so as to lock the cap 31 on the spigot 11 in a first position. If the projection 41 had been in the second circumferential slot 49, then the cap 31 would be locked onto the spigot 11 in a second position.

To release the cap 31, the cap 31 and barrel 3 are rotated in opposite projections to move the projection 41 out of the circumferential slot 47 or 49 and into the axial slot 45. In this position, the cap 31 can be pulled away from the barrel 3, sliding the projection 41 along and out of the axial slot 45, releasing the cap 31 from the spigot 11. Relocking the cap[ 31 on the spigot 11 is carried out by repeating the above actions in reverse. Two circumferential slots are provided since it is sometimes the case that, once the cap 31 has been removed, it cannot be pushed as far over the needle as was originally the situation and the second circumferential slot allows locking of the cap 31 even when the cap 31 has not been able to have been fully pushed home.

If it is desired to apply the lock to the embodiment of figure 4, it would be necessary to extend the larger diameter portion 37 of the cap 31 to provide a greater extension of the cap 31 lying over the barrel 3 and to form the projection 41 on the exterior of the barrel 3 instead of on the spigot 11.

It will be appreciated that modifications of or additions to the above described embodiments may be made without departing from the scope of the invention. For example, instead of having an increased spigot size as shown in figure 3, the cap could be formed with a reduced inner diameter potion at its open end to enable the cap to be of increased diameter generally without increasing the diameter of the spigot.

## Claims

1. A safety hypodermic syringe (1) comprising a barrel (3) having a plunger operated piston (17) therein, a hollow spigot (11) extending from one end (7) of the barrel (3) and connected to the interior of the barrel (3), an hollow needle (13) detachably attached to the spigot (11) by means of a cup portion (15) at the end of the needle (13) away from the point (18), the spigot (11) having a reduced diameter portion at its distal end for receipt of the needle cup portion (15) so that the cup portion (15) lies substantially flush with the remainder of the spigot (11) and a cap (31) detachably fitted to the said one end (7) of the barrel so as to cover the needle (13), wherein the cap (31) has a length (L) measured from the inner end of the needle (13) adjacent to the needle cup portion (15) to the opposite end of the cap (31), which is at least twice the length (I) of the needle (13) being the length of the needle (13) beyond the needle cup portion (15).

2. A safety hypodermic syringe as claimed in claim 1, wherein the inner diameter (D) of the cap (31) is at least one sixth larger than the outer diameter (d) of the needle cup portion (15).

3. A safety hypodermic syringe (1) comprising a barrel (3) having a plunger operated piston (17) therein, a hollow spigot (11) extending from one end (7) of the barrel (3) and connected to the interior of the barrel (3), an hollow needle (13) detachably attached to the spigot (11) by means of a cup portion (15) at the end of the needle (13) away from the point (18), the spigot (11) having a reduced diameter portion at its distal end for receipt of the needle cup portion (15) so that the cup portion (15) lies substantially flush with the remainder of the spigot (11) and a cap (31) detachably fitted to the said one end (7) of the barrel (3) so as to cover the needle (13), wherein the inner diameter (D) of the cap (31) is at least one sixth larger than the outer diameter (d) of the needle cup portion (15).

4. A safety hypodermic syringe as claimed in claim 2 or 3, wherein the inner diameter (D) of the cap (31) is at least 7 mm.

5. A safety hypodermic syringe as claimed in claim 4, wherein the inner diameter (D) of the cap (31) is at least 10 mm.

6. A safety hypodermic syringe as claimed in claim 4 or 5, wherein the inner diameter (D) of the cap (31) is at least 12 mm.

7. A safety hypodermic syringe as claimed in any one of claims 2 to 6, wherein the outer diameter of the spigot (11) is substantially the same as the inner diameter (D) of the cap (31).

8. A safety hypodermic syringe as claimed in any one of claims 2 to 6, wherein the cap (31) has an increased diameter at its open end to a diameter substantially the same as the outer diameter of the barrel (3) whereby the open end of the cap (31) can be seated on the end of the barrel (3).

9. A safety hypodermic syringe as claimed in any one of claims 2 to 8, wherein the length (L) of the cap (31) is at least twice the length (I) of the longest needle (13) usable in the syringe

10. A safety hypodermic syringe as claimed in any one of claims 2 to 8, wherein the length(L) of the cap (31) is at least 30 mm.

11. A safety hypodermic syringe as claimed in any one of claims 2 to 8, wherein the length (L) of the cap (31) is at least 44 mm.

12. A safety hypodermic syringe as claimed in any one of claims 2 to 8, wherein the length (L) of the cap (31) is at least 70 mm.

13. A safety hypodermic syringe as claimed in any preceding claim, wherein the barrel (3) and cap (31) have cooperating locking means (43) whereby the cap (31) can be locked in position relative to the barrel (3).

14. A safety hypodermic syringe as claimed in claim 13, wherein the spigot (11) has a radially outwardly extending projection(41) which cooperates with an axial slot (45) in the cap (31) extending in the axial direction of the cap (31) from the open end thereof.

15. A safety hypodermic syringe as claimed in claim 13 or 14, wherein a circumferential slot (49) is provided extending from the axial slot (45) and into which the projection (41) can be seated to stop removal of the cap.
